# EUROPEAN PATENT APPLICATION

(11) **EP 4 197 548 A1**
(43) Date of publication of application: **21.06.2023**
(21) Application number: 20934462.1
(22) Date of filing: 25.06.2020
(51) Int. Cl.: A61K 35/28, A61K 31/7105, A61P 31/14, A61P 29/00, A23L 33/10

(54) **COMPOSITION HAVING ANTI-INFLAMMATORY AND ANTIVIRAL EFFECT, COMPRISING PLACENTAL EXTRACELLULAR VESICLES**

(30) Priority: 04.05.2020 KR 20200053200; 19.06.2020 KR 20200074860
(71) Applicant: TS Cell Bio Co., Ltd., Wonju-si, Gangwon-do 26460 (KR)
(72) Inventor: MOON, Ji Sook, Seoul 05649 (KR)
(74) Representative: Danubia Patent & Law Office LLC
(86) International application number: PCT/KR2020/008277
(87) International publication number: WO 2021/225214

(57) **Abstract**

The present invention relates to kinds and effects of various materials derived from the placenta, in particular, kinds and effects of diverse biomolecules including: extracellular vesicle (EV) derived from the placenta, umbilical cord and/or cord blood, placental mesenchymal stem cell (pMSC) derived from the placenta, umbilical cord and/or cord blood, EVs derived from the above bio-materials, as well as miRNA and cargo contained in the above EVs. In this regard, miRNA contained in the EV efficiently degrades RNA genome of corona virus, thereby exhibiting direct anti-viral effects. Further, indirect effects of preventing and treating different virus infections owing to anti-inflammatory effects of various biomolecules inherently included in EV, can be identified, thereby completing the present invention.

## Description

### [Technical Field]

The present invention relates to kinds and effects of diverse substances derived from placenta and, more particularly, kinds and effects of various biomolecules including, for example, extracellular vesicle (EV) derived from placenta, umbilical cord and/or cord blood, placental mesenchymal stem cell (pMSC) derived from placenta, umbilical cord and/or cord blood, EVs derived from the above bio-materials, as well as miRNA and cargo contained in the above EVs. miRNA contained in the EV may efficiently degrade RNA genome of corona virus, thereby exhibiting direct anti-viral effects. Further, indirect effects of preventing and treating different virus infections owing to anti-inflammatory effects of various biomolecules inherently included in EVs, can be identified, thereby completing the present invention.

### [Background Art]

Placenta, umbilical cord and cord blood are fetus-derived tissues and stem cells obtainable during baby delivery and, in particular, placenta is a byproduct remaining after falling from the uterus of the mother, and may have a role of connecting the fetus and the body of the mother.

Till the present, the placenta, umbilical cord and cord blood have been recognized as temporary organs discarded after the delivery. However, it was currently discovered that diverse cells such as mesenchymal cells, deciduas cells, amnion, endothelial cells, etc. are present in different portions of the placenta.

Specifically, it is known that essential amino acids, melatonin, nucleic acid components such as RNA and DNA, antioxidant enzyme such as super-oxide dismutase (SOD), hyaluronic cid antioxidant, cytokine, an insulin-like growth factor, an epidermal growth factor (EGF) and a senescent cell activating factor (SCAF) are present in the placenta, umbilical cord and cord blood.

Further, it is known that the placenta, umbilical cord and cord blood are rich in nutrients to be provided to the fetus, while having immune-suppressing function to prevent the immune system of the mother from attacking the fetus.

In general, the placenta, umbilical cord and cord blood are used for diagnosis of the mother body or fetus, and then discarded. However, in order to utilize features of the placenta in medical applications, studies are continued in a plurality of fields.

From the time past in oriental medicine, the placenta is called "zahageo" and, when a body becomes weak, steamed placenta for eating was prescribed. Further, in order to use plenty nutrients in the placenta, cosmetics manufactured by extracting the nutrients from the placenta of animal and, similarly, a placenta nutrient injection manufactured by extracting the nutrients from the placenta of animal are now commercially available in the art.

In the medicine field, a medicament produced by hydrolyzing human placenta is on sale, for example, in an injection ample form. In particular, different products are developed with focusing on the nutrients of the placenta. However, studies make poor progress in aspects of extracellular vesicle (EV) derived from the placenta and various biomolecules, specifically, miRNA.

Meanwhile, the conventional virus treatment agents in clinical tests on progress till the present have involved strong side effects and are seldom prescribed for patients in mild cases or entailed limitations since it is difficult to administer a high content of anti-viral drug for treatment until desirable effects are expressed.

Accordingly, there is a requirement for developing many more virus-specific drugs to overcome side effects of the existing anti-virus agents.

### [Prior Art Document]

### [Patent Document]

(Patent Document 1) Korean Patent Registration No. 10-1555981

### [Disclosure]

### [Technical Problem]

The present disclosure has been proposed to overcome the aforementioned problems of the prior art, and an object of the present disclosure is to provide a composition with anti-inflammatory and anti-viral efficacy, by specifying and analyzing various biomolecules ("cargos") including extracellular vesicles (hereinafter, "EV") derived from the placenta, umbilical cord and/or cord blood, placental mesenchymal stem cells (pMSC) derived from the placenta, umbilical cord and/or cord blood, EVs derived from the above bio-materials, miRNA contained in the EVs, etc.

Another object of the present invention is to provide a composition for prevention or treatment of corona virus with frequent occurrence of variations, which binds to 3'UTR site with less mutation among genes of the corona virus.

### [Technical Solution]

According to an aspect of the present invention, a cell-free composition including at least one of carriers, excipients or diluents, as well as cargos extracted from the placenta, umbilical cord or cord blood.

The cargos described above may refer to any one or more selected from protein, peptide, antigen, antibody, protein fragment, DNA, RNA, cell, micro-vesicle and other bio-particles, preferably include extracellular vesicles (EVs) and miRNA, however, are not limited thereto.

In the present invention, the carriers, excipients and diluents may include, for example, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate and mineral oil.

The composition of the present invention is characterized by not including cells. Since embolism, hemo-coagulation and immune reaction may be caused if a stem cell is intravenously administered, the present invention may have an advantage in consideration of the above problem.

More particularly, the cargos may include EVs directly obtained from the placenta, umbilical cord and/or cord blood, or EVs obtained from placental mesenchymal stem cells (*"*pMSC*"*) which are derived from the placenta, umbilical cord and/or cord blood. The cargos may include, but not limited to, one or more selected from the group consisting of miR-92a-3p, miR-26a-5p, miR-23a-3p, miR-103a-3p and miR-181a-5p, etc., which are included in 77 miRNAs contained in tissue-derived EVs, tissue extract-derived EVs and pMSC-Evs.

Specifically, these 77 miRNAs may include hsa-miR-34a-5p, hsa-miR-197-3p, hsa-miR-92b-3p, hsa-miR-199a-3p, hsa-miR-181b-5p, hsa-miR-181a-5p, hsa-miR-1307-3p, hsa-miR-139-5p, hsa-miR-125b-5p, hsa-let-7a-5p, hsa-miR-100-5p, hsa-miR-26a-5p, hsa-let-7i-5p, hsa-miR-16-5p, hsa-miR-15a-5p, hsa-miR-92a-3p, hsa-miR-342-3p, hsa-miR-345-5p, hsa-miR-7-5p, hsa-miR-484, hsa-miR-328-3p, hsa-miR-22-3p, hsa-miR-324-3p, hsa-miR-744-5p, hsa-miR-451a, hsa-miR-423-5p, hsa-miR-423-3p, hsa-miR-193a-5p, hsa-miR-10a-5p, hsa-miR-21-5p, hsa-miR-3615, hsa-miR-320c, hsa-miR-122-5p, hsa-miR-24-3p, hsa-miR-27a-3p, hsa-miR-23a-3p, hsa-miR-150-5p, hsa-miR-99b-5p, hsa-miR-125a-5p, hsa-miR-26b-5p, hsa-miR-149-5p, hsa-miR-103a-3p, hsa-miR-99a-5p, hsa-let-7c-5p, hsa-miR-155-5p, hsa-miR-185-5p, hsa-miR-1249-3p, hsa-let-7b-5p, hsa-miR-425-5p, hsa-miR-425-3p, hsa-miR-191-5p, hsa-let-7g-5p, hsa-miR-15b-5p, hsa-miR-574-3p, hsa-miR-143-3p, hsa-miR-378a-3p, hsa-miR-146a-5p, hsa-miR-196b-5p, hsa-miR-25-3p, hsa-miR-93-5p, hsa-miR-29a-3p, hsa-miR-320a-3p, hsa-miR-30d-5p, hsa-miR-204-5p, hsa-let-7f-5p, hsa-let-7d-5p, hsa-let-7d-3p, hsa-miR-23b-3p, hsa-miR-27b-3p, hsa-miR-199b-3p, hsa-miR-126-3p, hsa-miR-221-3p, hsa-miR-222-3p, hsa-miR-532-5p, hsa-miR-223-3p, hsa-miR-652-3p and hsa-miR-424-3p.

Further, the cell-free composition of the present invention may include, but not limited to, one or more among EVs and/or the above 77 types of miRNAs as the cargos.

Micro-RNA (miRNA) may refer to a non-coding RNA in a small size which consists of nucleotides having a sequence length of 18 to 25 nucleotides, and miRNA was found to efficiently degrade RNA genome of SARS-CoV-2, thereby inhibiting virus.

The present inventors have discovered 77 miRNAs that directly react with 3'UTR of SARS-CoV-2, and are present in tissue-EVs, tissue extract-EVs and MSC-EVs. Thereamong, 18 miRNAs bound to 3'UTR of SARS-CoV-2 are selected. Furthermore, top five (5) miRNAs having higher expression level and stronger binding ability are chosen from the above selected miRNAs.

Specifically, these 18 miRNAs may include hsa-miR-92a-3p, hsa-miR-92b-3p, hsa-miR-181a-5p, hsa-miR-26a-5p, hsa-miR-34a-5p, hsa-miR-23a-3p, hsa-miR-125b-5p, hsa-miR-125a-5p, hsa-miR-103a-3p, hsa-miR-223-3p, hsa-miR-25-3p, hsa-miR-26b-5p, hsa-miR-193a-5p, hsa-miR-1307-3p, hsa-miR-155-5p, hsa-miR-185-5p, hsa-miR-424-3p and hsa-miR-23b-3p. Further, the top five (5) miRNAs chosen among the same may be 103a-3p, miR-181a-5p, miR-26a-5p and miR-23a-3p.

In the embodiments of the present invention, it was found that the five (5) miRNAs are all expressed from tissue-EVs and/or tissue extract-EVs, thereby successfully inhibiting SARS-CoV-2.

Further, tissue-EVs, tissue extract-EVs and placental mesenchymal stem cell-derived vesicles (pMSC-EV) existing in MSC-EVs may include many anti-inflammatory factors known to prevent fatal cytokine storm and have excellent regeneration effects. As a result of experiments, it was confirmed that EV controls inflammatory environments in some host cells known to express ACE2 receptor so as to inhibit inflammation reaction.

According to another aspect of the present invention, an anti-viral composition including the cell-free composition as described above may be provided.

More particularly, the virus may include, but not limited to, one or more selected from the group consisting of corona virus, HIV, influenza, entero-virus, Porcine reproductive and respiratory syndrome virus, C type hepatitis virus and Bovine viral diarrhea virus, more specifically, NMC-nCoV02 or SARS-CoV-2.

According to another aspect of the present invention, a pharmaceutical composition for prevention or treatment of viral infection, including the anti-virus composition as described above, may be provided.

Specifically, the virus may include, not limited to, corona virus.

The pharmaceutical composition may further include any carrier, excipient and diluents used for general pharmaceutical compositions.

The carrier, excipient and diluents may include, but not limited to, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate or mineral oil.

Further, the composition may be formulated into an oral formulation such as dispersions, granulates, tablets, capsules, suspensions, emulsions, syrups, aerosols, etc., or a parenteral formulation such as injection, nasal spray, inhalation liquid, cream, gel, ointment, suppository or lotion for use.

A solid type formulation for oral administration may include, but not limited to, tablets, pills, dispersions, granulates, capsules, etc. wherein the solid formulation may be prepared by mixing the miRNA composition and fractions thereof with at least one of excipients, for example, starch, calcium carbonate, sucrose, lactose or gelatin. Other than the excipients, a lubricant such as magnesium stearate, talc, etc. may be used.

The liquid formulation for oral administration used herein may include suspensions, oral liquids, emulsions, syrups, etc. Further, other than simple diluents such as water and liquid paraffin, a variety of excipients such as wetting agents, sweeteners, flavoring agents, preservatives, etc. may also be included.

Formulations for parenteral administration used herein may include sterilized aqueous solution, non-aqueous solvents, suspensions, emulsions, lyophilized formulations, suppository agents, etc. The non-aqueous solvents or suspensions used herein may include propylene glycol, polyethylene glycol, vegetable oil such as olive oil, injectable ester such as ethyl oleate, etc. Meanwhile, a basic material of the suppository agent may include witepsol, macrogol, tween 61, cacao butter, laurin butter and glycerol-gelatin.

A pharmaceutical composition including the anti-virus composition may be administered in a pharmaceutically effective amount to a subject to be treated. The *"*pharmaceutically effective amount" means an amount sufficient to treat a disease in a rational benefit/danger ratio applicable to medical treatment, while an effective dose level may be determined based on: types of diseases of patient; severity; activity of drug; sensitivity to drug; administration time; administration route and emptying rate; treatment period; factors including other drugs to be simultaneously used; and other factors well known in medical applications.

The pharmaceutical composition may be administered as an individual treatment agent or in combination with other treatment agents. Further, this composition may be administered along with any conventional treatment agent successively or simultaneously. Further, the composition may be administered in a single time or in multiple times. In consideration of all of the above factors, the composition is preferably administered in a minimum amount with which maximum effects can be obtained without adverse effects. The amount may be easily determined by those skilled in the art.

A preferable amount of the composition for treatment according to the present invention may vary according to condition and weight, severity of disease, drug type, administration route and administration period, however, can be suitably selected by those skilled in the art to which the present invention pertains. Preferably, the composition for treatment according to the present invention may be administered such that the number of EVs reaches 10² to 10¹⁶, more preferably, 10⁵ to 10¹² per day with reference to an amount of EV, but not limited thereto.

Further, with reference to an amount of miRNA, the administration amount may range from 0.001 to 1000 mg/kg, preferably, 0.01 to 100 mg/kg in order to attain more improve effects. The administration may be conducted once or separately conducted in several times a day. The administration amount and the administration frequency do not limit the scope of the present invention in any aspect.

According to another aspect of the present invention, there is provided a food composition for prevention or improvement of viral infection, which includes the anti-virus composition.

Since the anti-virus composition of the present invention includes extracellular vesicles (EV), even when the composition is orally taken, EV and miRNA may be delivered into the body, thereby exhibiting excellent effects of preventing and treating viral infection.

In the present invention, food refers to natural or processed products containing one or more nutrients, preferably means a condition thereof that is obtained through some processing stages to be directly edible by a human or animal. Commonly, this meaning intends to include all of beverages, food additives and drink additives.

The food of the present invention may include, for example, various foods, beverages, gum, tea, etc. The above foods, beverages, food additives and drink additives may be produced by conventional manufacturing processes.

In the present invention, beverage generally refers to all of drinks in order to slake thirst or enjoy taste, and also intends to include health functional beverages. The beverage includes an essential component, that is, the composition of the present invention as an active ingredient in a predetermined ratio, while other ingredients may also be included without particular limitation thereof. like typical beverages, different flavoring agents or natural carbohydrate may also be included as additional components.

The natural carbohydrate may include general sugars, for example, monosaccharide such as glucose or fructose, disaccharide such as maltose, sucrose, etc., polysaccharides such as dextrin, cyclodextrin, etc., as well sugar alcohol as xylitol, sorbitol, erythritol, etc. Other than the above materials, flavoring agents including natural flavoring agents (thaumatin, stevia extract (for example, rebaudiocide A, glycyrrhizin, etc.) and synthetic flavoring agents (saccharine, aspartame, etc.) may be advantageously used. A ratio of the natural carbohydrate may be generally about 1 to 20 g, preferably 5 to 12 g per 100 ml of the composition of the present invention. Furthermore, the composition of the present invention may further contain fruit pulp for production of natural fruit juice, fruit juice beverage, vegetable beverages, etc.

Moreover, the food composition of the present invention may further include various nutritional medicines, vitamins, minerals (electrolytes), flavoring agents such as synthetic flavoring agents and natural flavors, colorants and promoters (cheese, chocolate, etc.), pectic acid and salts thereof, organic acid, protective colloids, thickeners, pH adjusters, stabilizers, preservatives, glycerin, alcohol, carbonating agents for carbonated drinks, or the like. These components may be used alone or in combination with one or more thereof.

### [Advantageous effects]

The present invention provides extracellular vesicles (EV) derived from the placenta, umbilical cord and/or cord blood, placental mesenchymal stem cell (pMSC) derived from the placenta, umbilical cord and/or cord blood, and a composition including the same. Further, when developing a therapeutic agent of preventing or treating corona virus using the composition: 1) miRNA of the present invention is combined with 3'UTR of a corona virus genome at high specificity, therefore, it is presumed that very little side effects on normal tissues and organs are caused; 2) 3'UTR of the corona virus genome is a part highly retained with very little mutation, this part is less influenced by mutation even in RNA virus with frequent mutation, which in turn is generally used for RNA virus; and 3) diverse bio-molecules existing in EV have additional effects such as regeneration of damaged tissues and immune adjustment, therefore, it is presumed that any therapeutic agent using the same exhibits very excellent effects.

Further, in addition to the direct anti-viral effects described above, the composition of the present invention may include different bio-materials inhibiting inflammation through interaction with gene associated with inflammation as well as miRNA in EV, thereby exhibiting indirect effects of alleviating symptoms caused by viral infection.

In other words, the composition of the present invention may show both of direct virus inhibitory effects and indirect therapeutic effects to inhibit inflammation caused by viral infection and cytokine storm, thereby being useable as a prophylactic agent for viral infection.

Functional effects of the present invention are not particularly limited to the above effects, instead, it should be construed as including all effects presumable from the configurations of the present invention disclosed in the detailed description and the appended claims of the present invention.

### [Description of Drawings]

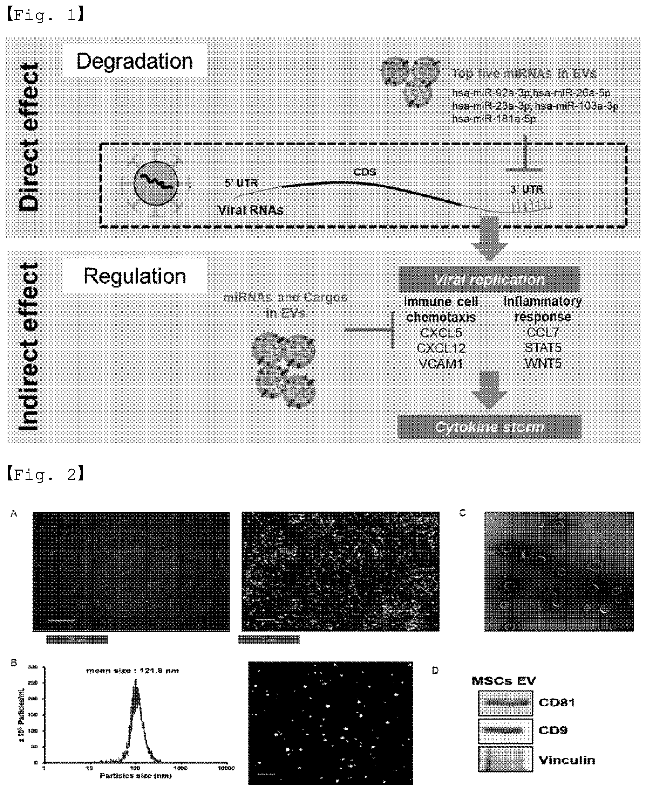

FIG. 1 illustrates a functional mechanism for anti-viral effects of tissue-EV, tissue extract EV, MSC-EV and miRNA included therein with regard to SARS-CoV-2 virus, and it could be seen that direct anti-viral effects include down control of SARS-CoV-2RNA induced by direct binding of miRNA in sites such as 3'UTR, 5'URT or a coding sequence of SARS-CoV-2, while indirect anti-viral effects include adjustment of inflammatory mRNA expression through miRNA and protein inherent in EV so as to regenerate damaged tissues, while controlling anti-inflammatory circumstances through miRNA and protein of the regenerated tissues so as to inhibit cytokine storm.
FIG. 2 illustrates observed results of pMSC-EV characteristics (A: pMSC-EV is positive for CD63 measured by fluorescence microscopy. B: an average size of pMSC-EV is 121.8 mm in diameter for 1 ml of extract. C: Representative TEM image. D: Western blott's results of pMSC-EV with regard to CD81, CD9, annexin A2 and HS970) .
FIG. 3 illustrates a potential binding site at which miRNA of the present invention can be bound to 3'UTR of SARS-CoV-2.
FIG. 4 illustrates results of analyzing qPCR in order to confirm whether miRNA of the present invention exists in EV.
FIG. 5 illustrates results of luciferase analysis in order to identify direct anti-viral effects of EV according to the present invention (A: a recombinant plasmid prepared by cloning a PCR fragment to a luciferase reporter plasmid between luciferase ORF and the synthetic poly (A) sequence. B: activity of opposite luciferase in SK-N-BE(2)C cells transfected with the recombinant plasmid).
FIG. 6 illustrates results of confirming whether miRNA of the present invention is effective for various corona viruses (A: a binding site of miRNA as well as 3'UTR of each of five corona viruses. B: interaction between miRNA of the present invention and other viruses).
FIG. 7 schematically illustrates an experimental method for identification of indirect anti-virus effects of EV and miRNA, respectively.
FIG. 8 illustrates results of confirming effects after treatment of BEAS-2B (gastrointestinal ephithelia) with EV and miRNA (A: EV, B: miRNA).
FIG. 9 illustrates results of confirming protection and recovery effects when treating LL-24 with EV and miRNA (A: EV, B: miRNA).
FIG. 10 illustrates results of confirming protection and recovery effects of MEF (fibroblast) and BV2 (microglial cell line), in particular, in FIG. 10a and 10b, respectively.
FIG. 11 illustrates results of confirming expression levels of inflammation relevant factors after treatment of neuron cells, M2, with EV.
FIG. 12 shows photographed images before and after treatment of each of cell lines with PKH26-labeled EV.
FIG. 13 is a conceptual view illustrating viruses and processes of treating or improving viral infection symptoms with EV and miRNA of the present invention through direct and indirection routes.

### [Detailed Description of Preferred Embodiments of Invention]

Hereinafter, the present invention will be described in detail by means of preferred embodiments. However, the following embodiments are proposed for illustrative purposes only and the present invention is not particularly limited thereto.

### Experimental method and material

### Preparation of placenta extract and EV

Human placenta not involving symptoms of internal, obstetrical or surgical complication has been obtained. Each placenta was carefully incised, washed with PBS several times, mechanically pulverized, and digested with 0.5% collagenase IV (Sigma, St. Louis MO, USA) at 37 °C for 30 minutes to obtain the placenta extract and EV.

### Cell line and virus

A cell line of renal epithelial cells of African green monkey, that is, vero cells were used. The cells were incubated in DMEM medium (Dulbecco's Modified Eagle's medium, Gibco, USA).

In early February in 2020, corona-19 virus (NMC-nCoVO2) and SARS-CoV-2 virus separated from the respiratory system specimens of domestic infected patients were used for infection of vero E6 cells, and a virus titer was determined by 50% tissue culture infection dose (TCID₅₀) with regard to cytopathic effects (CPE). Further, all experiments for viruses were implemented in biological safety level-3 (BLS-3) laboratories.

### Assessment of cytotoxicity

Cytotoxic effects of EV to vero cells were evaluated by MTT-assay.

More particularly, a vero cell layer in 96-well plate was washed with phosphate buffer saline (PBS) and treated with a continuous amount of EV. 1 × 10⁴ vero cells were incubated in 96-well plate at 37 °C under 5% CO₂ for 24 hours. Absorbance was measured by a plate reader at a wavelength of 500 to 600 nm.

### Assessment of inhibitory effects of cytopathic effects (CPE)

After introducing 1 × 10⁴ vero cells in 96 wells, using 50 µL of culture solution containing EV and 50 µL of solution containing SARS-CoV-2 virus were treated at 37°C, respectively. The cells inoculated with virus was used as a control. The infected cells were observed with 100% CPE under a microscope. The cells were stained with 1% crystal violet. The percentage of CPE in EV-treated cells was calculated (positive well/total well), and a result thereof was photographed.

### Nano-particle tracking and analysis

EV was diluted into 1 mL with PBS and inspected by a ZetaView nanoparticle tracking video microscope (Particle Metrix, Innig, Germany). A basic setting for EV or nanoparticles by a software manufacturer was chosen and, for each measurement, scanning was conducted three times.

### Fluorescence imaging of EV

EV was biotinylated using EZ-Link Sulfo-NHS-LC-LC-Biotin (Thermo Fisher Scientific, Waltham, MA, USA).

The biotinylated EV was loaded to Zeba spin desalting columns 7K MWCO (Thermo Scientific), followed by removing the remaining free biotin.

For staining, 20 µL of biotinylated EV was added to a circle drawn on a streptavidin coated glass slide (Arrayit Corporation) and, after 30 minutes, EV was fixed with BD Fix Perm (BD bioscience), followed by blocking the same with 0.2% BSA-PBS.

EV was subjected to immune fluorescent staining using anti-human CD 63 (1:100, Santa Cruz Bio-Technology in Dallas, Texas, USA, (SC-5275)) at room temperature for 2 hours, followed by incubation along with a secondary antibody conjugated with Alex Fluor 488 at room temperature for 1 hour.

### Transmission electron microscopic (TEM) imaging

5 ml of aliquot of the diluted sample containing 0.5 µg of protein was dropped on a hydrophilic grid. After several minutes, the grid was washed with distilled water and compared with 2% uranyl acetate for 20 seconds. Under JEM-1010 microscope (JEOL) operating at 80 kV, images were acquired.

### Western blotting

EV was dissolved in 10 x RIPA buffer containing protease cocktail tablets (Roche) and phosphatase inhibitors II and III (Sigma). According to BCA assay (Thermo Fisher Scientific), a protein concentration was determined. After adding 10% SDS-PAGE to 30 µl aliquot of each sample, western blotting was conducted.

The blotting was blocked in 10% skim milk of TBS-T. The following antibodies to protein were obtained from the following suppliers: CD81 (1:1,000, Santa Cruz Biotechnology), Alix (1:1,000, Santa Cruz Biotechnology), CD9 (1:500, Santa Cruz), and GAPDH (1:10,000, sc-32233, Santa Cruz Biotechnology).

A primary antibody was diluted in TBS-T and incubated along with blotting at 4 °C, followed by incubation using a secondary antibody for 1 hour. Using an enhanced chemiluminescent HRP substrate (Millipore), immune response was detected.

### Cell culture

A human liver cancer cell line HepG2 and a mouse microglial cell line BV2 were purchased from American Type Culture Collection (ATCC, Manassas, CA, USA). Each of these cell lines was cultured in a growth medium DMEM (Gibco, Carlsbad, CA, USA) supplied with 10% fetal bovine serum (FBS, Gibco) and 1% penicillin/streptomycin (P/S) at 37 °C under CO₂. Both cell lines were sub-cultured by 2 to 4 days.

A normal lung cell line LL24 was also obtained from ATCC. LL24 cells were grown in a RPMI-1640 medium supplemented with 10% FBS and 1% P/S (Gibco/Life Technologies, USA, NY, USA) at 37 °C under 5% CO₂ humidified atmosphere. Sub-culture was executed at an interval of 3 days.

MEF cells were isolated from mouse fetus on 13.5 days. The MEF cells were cultured in a DMEM medium supplemented with 10% FBS, 1% antibiotics and antifungal agent (250 ng/mL amphoterisin, 100 U/mL penicillin and 100 ug/mL streptomycin), 8 ug/ml tyrosin (Sigma) and 15 ug/mL gentamicin (Gibco) at 37 °C under 5% CO₂. The MEF cells were sub-cultured at an interval of 3 days and used in 2^{nd} to 5^{th} subcultures.

Transformed human bronchial epithelial cell line BEAS-2B was cultured in 5 ug/mL of gentamicin (Gibco) and non-serum 1 X defined keratinocyte SFM (Gobco) at 37 °C under 5% CO₂. The medium was changed at an interval of 2 to 3 days and the cells were sub-cultured at an interval of 4 to 5 days.

Neurons were isolated from human fetal tissues at 10 weeks, 12 weeks and 14 weeks after pregnancy with the consent of the mothers. The neurons were cultured in a DMEM/F12 medium (Gibco) supplemented with 5 ug/mL of a supplement, that is, B27 (Gibco), gentamicin (Gibco), human bFGF, 20 ng/mL of human EGF (Peprotech), 1 ug/mL of pocopherol and tocopherol acetate (Sigma Aldrich) at 37 °C under 5% CO₂ and 3% O₂.

### EV labeling with PKH26

EV was labeled by a PKH26 red fluorescent cell linker kit for general cell membranes (Sigma-Aldrich).

Specifically, after re-suspending 200 µl/mL of EV pellets in diluents C, the solution was mixed with a dye solution (2 µL of PKH26 ethanol dye and 500 µL of diluents C) in 1:1 ratio for 5 minutes. Next, the same volume of 1% bovine serum albumin (BSA) was added thereto in order to combine EV with an excess of dye, followed by treating the produced solution in 7K MWCO Zeba spin desalting column (Thermo Fisher Scientific) in order to remove the excess of dye. Thereafter, the sample was stored at -80 °C.

### LPS and EV treatment

For experiments, the cells were seeded with the same density in each well of 96-well or 6-well plate (Nunc, Roskilde, Denmark). In order to determine recovery effects, the cells were stimulated with LPS (MEFs and BV2: 0.5 to 2 µg/mL, HepG2 and LL24: 2 to 4 pg/mL), followed by EV treatment for 24 hours.

In order to determine protection effects, the cells were pre-treated with EV for 24 hours, and then, stimulated with LPS for 24 hours. After incubation for 24 hours, the cells were subjected to MTT assay or RNA extraction.

### miRNA transfection

in order to transfect miRNA, 6 × 10⁴ to 2 × 10⁵ cells were seeded in a 24-well plate, followed by infection with 20 nM miRNA (hsa-miR-92a-3p, hsa-miR-26a-5p, hsa-miR-23a-3p, hsa-miR-103-3p, and hsa-miR-181-5p) (Bioneer). As a Pre-miR miRNA negative control, lipofectamin 3000 was used.

### MTT assay

EV cytotoxicity was assessed by MTT assay. Briefly, the cells were seeded in a 96-well plate, treated with EV at a predetermined concentration, and stimulated with LPS for 24 hours. After removing the culture supernatant, the produced dark blue crystals were dissolved in DMSO. Absorbance was measured at 570 nm.

### RNA extraction and quantitative PCR (qPCR)

Total RNAs for qPCR was extracted from BV2, MEF, HepG2, LL24, BEAS-2B and neuron cells, while miRNA was extracted from EV. Extraction of total RNA was implemented using TRIzol reagent (Invitrogen).

Using cDNA kit (iNtRON), cDNA was synthesized from 1 µg of total RNA. Total exosome RNA and a protein separation kit (Thermo Fisher Scientific) were used to extract miNRA, followed by reverse transcription using miScript II RT (Qigen).

For PCR and qPCR, a primer of gene associated with inflammation was designed. A reaction mixture (total volume 20 µL) contained 10 µM primer mixture, SYBR-green (Enzynomics), water without nuclease (dark, nonionic) and 2 µL of cDNA. Conditions are as follows: denaturalization/activation stage was conducted at 95 °C for 10 minutes, followed by repeating 40 cycles at 95°C for 15 seconds, at 56 °C for 30 seconds and at 72 °C for 20 seconds. The reaction was conducted by StepOne Real Time PCR instrument (Applied Biosystems). Quantification of gene expression was based on C_{T} value of each sample.

### Transfection and reporter assay

208 bp fragment of 3'UTR of SARS-CoV-2 genome was synthesized by Bionics. 3'UTR fragment was degraded with Xba I, inserted at the downstream of firefly luciferase gene in pGL3-control (Promega), thereby obtaining pGL3covi-3UTR-Luc.

All constructions were confirmed by sequencing. Human neuroblastoma SK-N-BE(2)C cells were maintained in a DMEM supplemented with 10% FBS. All culture media included 100 U/mL penicillin and 100 mg/mL streptomycin. For transfection, the cells in no-antibiotics DMEM were plated in 24-well plate with 1.2 x 10⁵ cells/well 1 day before transfection. The transfection was implemented using lipofectamine 2000 (Invitrogen). A total amount of DNA was 0.5 µg/well, which comprised 0.2 µg of pGL3covi-3UTR-Luc and 0.3 µg of pRSV βgal as internal controls. Each transfection also contained labeled miRNA. 24 hours after transfection, cells of each well were dissolved in 100 µL dissolution buffer (25 mM tris-phosphate [pH 7.8], 2 mM DTT, 2 mM CDTA [1,2-diaminocyclohexane-N,N,N',N'-tetraacetic acid], 10% glycerol and 1% triton X 100). The same volume of firefly luciferase substrate was added thereto, lucifease activity was measured by means of a luminoometer plate reader, followed by normalization with regard to beta-galactosidase.

### RNA sequencing and data process

A small RNA sequence analysis (*"*sequencing*"*) was performed using extracellular vesicles (EV) obtained from two placental tissue extracts as well as a hpMSC medium under eight (8) different conditions. The sequencing was implemented by BGISeq-500 in Bajing Gene Institute (BGI, Shenzhen, China). Reads were aligned on human reference genome (GRCh38) using a subread aligner, and a featureCounts tool was used to obtain miRNA read counts. The number of reading miRNA was standardized, and low expressive miRNA was filtrated by means of edger R package. Each miRNA expression was modified into CPM (counts per million). Reading quality was managed using qrqcR package, and a distribution of small RNA frequency was acquired using sRNA toolbox.

### miRNA target estimation and analysis of function

In order to investigate a miRNA binding site in 3'UTR, PITA tool was used (Kertesz et al., 2007).

SARS-CoV-2 complete genome sequence (NC_045512.2) was obtained from NCBI standard sequence database (O*'*Leary O'Leary et al. 2016), and 3'UTR sequence was extracted from SARS-CoV-2 complete genome.

In order to expect a binding site of miRNA, a PITA tool with basic setting values was used.

A new miRNA binding site was estimated by miRDB user designated estimation tool (Chen and Xiaowei, 2020).

In order to determine biological functions of miRNAs, experimentally verified target of miRNAs was obtained from miRTarBase (Huang et al., 2020), miRNA to reach a rate of less than 1% was excluded from the analysis.

In order to investigate biological functions of miRNA, DAVID Bioinformatics Resource 6.8 (Huang et al., 2009) was used to perform GO term and KEGG pathway analysis.

Analyzed results of the function were visualized by Cytoscape 3.8 and Pathview R package.

### ACE2 expression assay

Human tissue and cell line expression data was acquired from Human Protein Atlas database (Uhlen et al., 2015). Using the expression data, ACE2 expression of each cell or tissue type was investigated.

### Analysis of preserved miRNA binding site

In order to investigate the preserved region of miRNA binding site in SARS-CoV-2, 3'UTR sequence was obtained from NCBI reference sequence database, and a virus 3'UTR sequence relevant to SARS-CoV-2 was used.

Specifically, SARS corona virus (NC_004718.3), SARS-CoV-2 (NC_045512.2), SARS corona virus BJ01 (AY278488.2), bat SARS corona virus HKU3-1 (DQ022305.2), bat SARS-like corona virus SL-CoVZC45 (MG772933.1) and bat SARS-like corona virus SL-CoVZXC21 (MG772934.1) were used.

The 3'UTR sequence of the corona virus was aligned by MUSCLE tool (Edgar et al., 2004), and MUSCLE assay adopted a basic value.

### Statistical assay

The statistical assay was performed according to ANOVA and HSD post-test of Tukey in R. Hit-map was created by expressing log12-CPM of miRNA in hpMSC-EV and using gplots R package (Warnes et al., 2015). P value in GO term assay and KEGG route assay was amended for multiple comparison using Benjamini-Hochberg method. Data was proposed by mean ± standard error, and p value or adjusted p value < 0.05 was considered to be significant.

Hereinafter, the present invention confirmed by the above-mentioned experimental method will be described in detail along with the accompanying drawings in terms of effects of the preset invention. However, such a description is proposed for helping understanding of the present invention, and the present invention is not restricted by these embodiments.

### Example 1. Virus inhibition mechanism of placenta-EV miRNA to SARS-CoV-2 virus

FIG. 1 illustrates a functional mechanism for anti-viral effects of placenta-EV miRNA with regard to SARS-CoV-2 virus. Direct anti-viral mechanism is formed by directly binding miRNA in a region such as 3'UTR, 5'URT or a coding area of the virus so as to induce down control of SARS-CoV-2 RNA. Further, indirect anti-viral effects include adjustment of inflammatory mRNA expression through miRNA so as to inhibit cytokine storm.

In other words, according to the present invention, it was confirmed that a mesenchymal stem cell (MSC)-derived EV and miRNA and protein molecules included therein may regenerate damaged tissues, while controlling miRNA and anti-inflammatory circumstances.

EV separated from pMSC was positive to a typical EV marker, that is, CD63 (FIG. 2a). A size of EV was analyzed nanoparticle tracking assay (NTA) and transmission electron microscope inspection (TEM).

A hydraulic diameter of exosome measured by the nanoparticle tracking assay (NTA) is 121.8 nm (FIG. 2b), and representative TEM image thereof is shown in FIG. 2c. Additional features of EV separated by western blotting of EV marker are those of typical EV markers such as CD81, CD9, annexin A2 and HSP70 (FIG. 2d).

### Example 2. Selection of miRNA bound to SARS-CoV-2 virus

On the ground that virus genome could become a target by human miRNA, the present inventors confirmed whether miRNA in EV may have interaction with 3'UTR of SARS-CoV-2 or not.

In SARS-CoV2 3'UTR, binding sites to miR-92a-3p, miR-26a-5p, miR-23a-3p, miR-103a-3p and miR-181a-5p are included.

Specifically, 3'UTR sequence of SARS-CoV-2 was aligned and the sequence was analyzed using MicroInspector software. As a result, total 18 miRNAs targeted 3'UTR of SARS-CoV-2 according to PITA software (Table 1), and it was expected that 27 miRNAs were bound to the entire genome site of SARS-CoV-2 virus (Table 2).

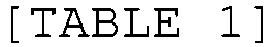

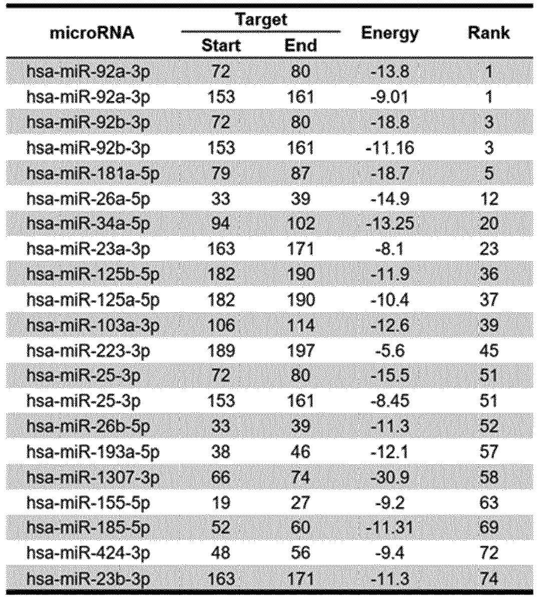

Table 1 shows a list of 18 miRNAs targeting 3'UTR of SARS-CoV-2 virus.

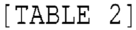

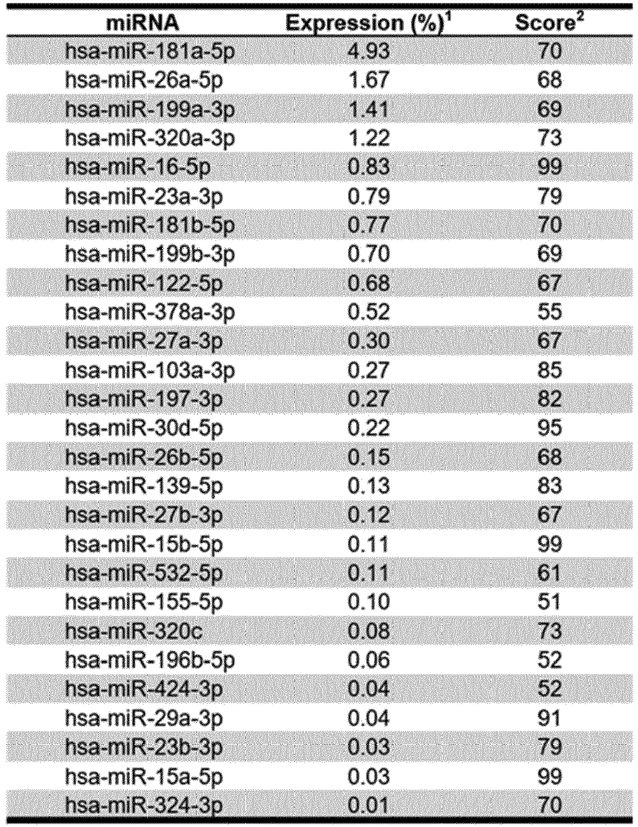

Table 2 shows a list of 27 miRNAs expected to be bound to the entire genome site in SARS-CoV-2 virus. In Table 2, expression (%) was calculated by the corresponding miRNA CPM/total miRNA CPM, wherein with binding possibility will increase with higher score. According to referential literature, when the score is 80 or more, the binding is possible.

Table 3 below shows information on the binding site in SARS-CoV-2 genome and results of binding prediction. 5 miRNAs (miR-92a-3p,miR-26a-5p, miR-23a-3p, miR-103a-3p, miR-181a-5p) were selected based on thermodynamic energy score and high scores of two prediction tools, that is, PITA and miRDB.

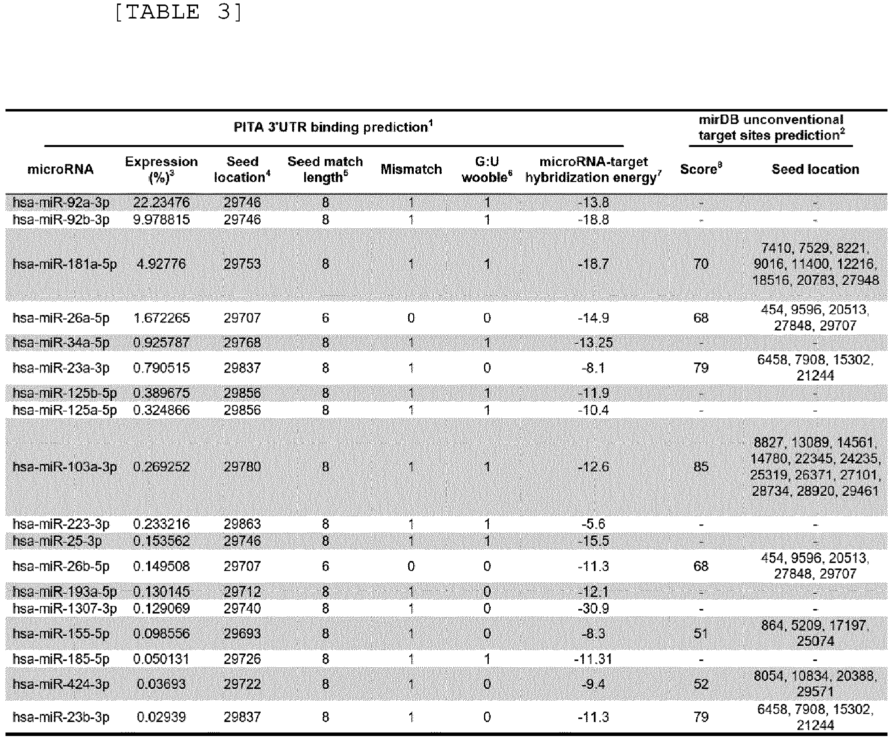

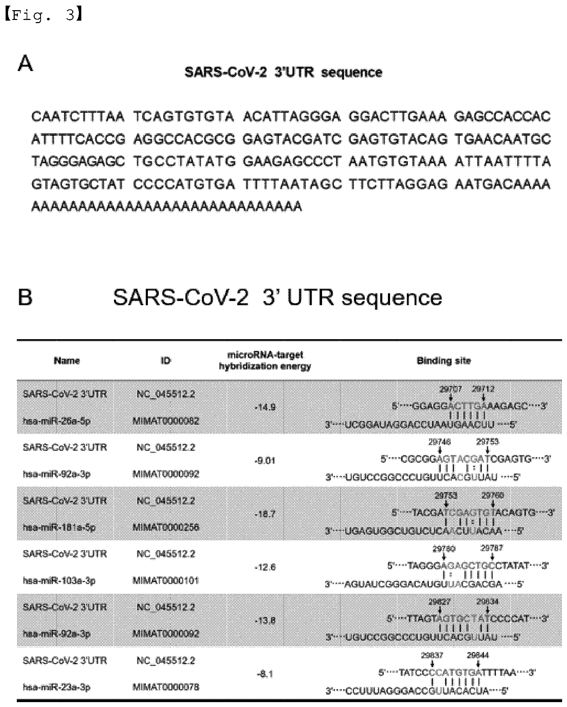

Using PIA, it was predicted that potential binding sites at which finally selected five (5) miRNAs, that is, miR-92a-3p, miR-26a-5p, miR-23a-3p, miR-103a-3p and miR-181a-5p may be bound to 3'UTR of SARS-CoV-2 (FIGS. 3a, 3b). Using PITA, total free energy was obtained for each of the miRNAs. For example, the binding energy of miR-181a-5p to 3'UTR is microRNA target hybridization energy of -18.7 kcal/mol. This suggests that miRNA 3'UTR binding would be voluntarily performed. Low binding thermodynamic energy (kcal/mol) indicates stronger binding.

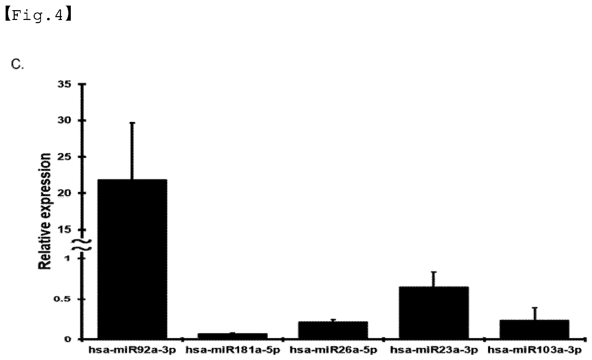

Next, in order to confirm whether the above 5 miRNAs exist in EV, qPCR analysis was implemented (FIG. 4). As a result, it was found that miRNAs in EV were highly expressed in the order of miR-92a-3p, miR-181a-5p, miR-26a-5p, miR-23a-3p and miR-103a-3p. That is, the expression of miR-92a-3p and miR-181a-5p was significantly higher in the same order as the predicted values. These results suggest that EV includes 5 miRNA, and therefore, can be used by itself as a therapeutic agent for SARC-CoV-2.

### Example 3. Confirmation of direct anti-viral effects of EV and miRNA

In order to confirm whether the specific miRNAs selected in Example 2 alone or 5 miRNAs can directly interact with SARS-CoV-2 genome, luciferase assay was implemented.

According to luciferase-reporter assay, whether the specific miRNAs or 5 miRNAs separately could direct interact with SARS-CoV-2 genome was confirmed.

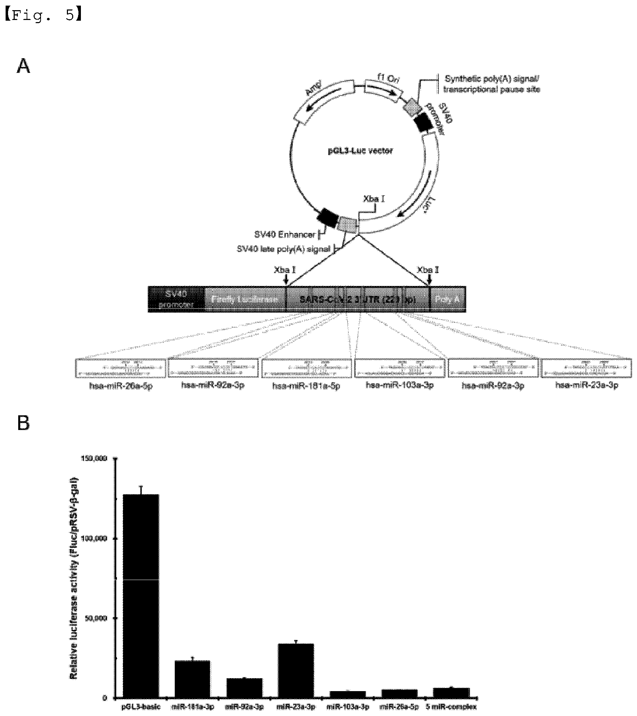

PCR fragments were cloned to the luciferase reporter plasmid between luciferase ORF and synthetic poly(A) sequence. The recombinant plasmid was named pGL3 SARS-CoV-3'-UTR_Luc (FIG. 5a). Then, the recombinant plasmid was transfected to human neuroblastoma SK-N-BE(2)C cells and, 48 hours after transfection, luciferase activity was measured.

As shown in FIG. 5b, relative luciferase activity in SK-N-BE(2)C cells transfected with the recombinant plasmid was considerably reduced as compared to cells transfected with empty psi-control vector (ps < 0.0001).

Specifically, the relative luciferase activity was down controlled when 3'UTR of the corona virus was co-transfected to DF-1 cells by 5 miRNAs or expression vectors thereof. From the results, it was confirmed that luciperase activity of psi-59UTR was considerably reduced.

The above experimental results indicate that miR-92a-3p, miR-26a-5p, miR-23a-3p, miR-103a-3p and miR-181a-5, respectively, are bound to 3'UTR of SARS-CoV-2 so as to induce significant silence.

Based on the above results, it was confirmed that all of the above 5 miRNAs may be directly bound to 3'UTR of SARS-CoV-2, and at least one miRNA selected from the group consisting of miR-92a-3p, miR-26a-5p, miR-23a-3p, miR-103a-3p and miR-181a-5p may degrade SARS-CoV-2 virus genome, thereby inhibiting SARS-CoV-2 infection.

### Example 4. Confirmation of direct anti-viral ability to different corona viruses and other viruses

In order to identify an assumption (or a hypothesis) that 3*'*UTR sequence would be preserved throughout corona virus family and a miRNA target site would be preserved over all of corona viruses, 5 corona viruses were randomly chosen and sequences in the 3'UTR region were compared by MUSCLE tool.

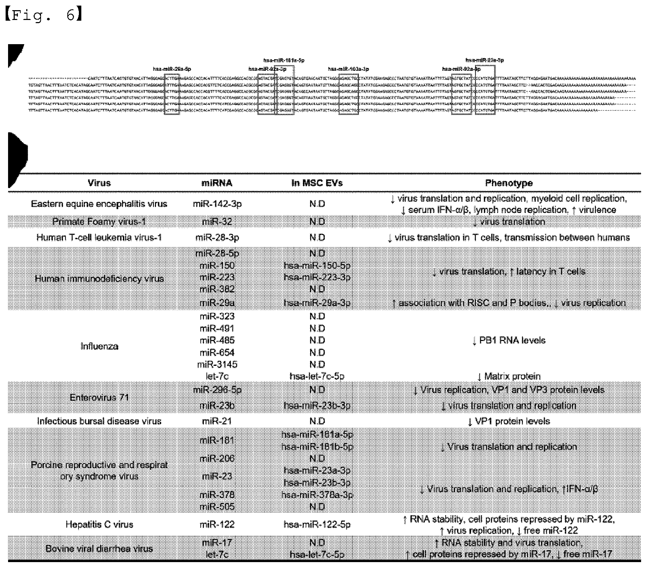

As shown in FIG. 6a, 3'UTRs of five (5) corona viruses were mostly preserved to show only small variation. In particular, binding sites predicted to be combined with 5 miRNAs (indicated by red box) were also confirmed to be preserved very well.

In other words, as a result of confirming 3'UTRs of various corona viruses, very little mutation occurred in 3'UTR. Further, in consideration of high miRNA binding rate, it is understood that 5 selected miRNAs may have general virus inhibitory ability applicable to even newly generated viruses by mutation.

Further, it is already known that different miRNAs in EV may interact with other viruses (FIG. 6b).

Specifically, it was demonstrated that miR-150-5p, miR-223-3p and miR-29-3p may interact with HIV, thereby inhibiting virus translation and final latency T cells.

It is known that miR-23b-3p may block translation of entero-virus 71 while Let-7c-5p may reduce a significant matrix protein for influenza virus.

miR-181a-5p, miR-181b-5p, miR-23a-3p, miR-23b-3p and miR-378a-3p may degrade porcine reproductive and respiratory syndrome virus.

miR-122-5p and let-7c-5p may inhibit C type hepatitis virus and bovine viral diarrhea virus, respectively.

In other words, EV and different miRNAs included in EV may be bound to various viruses and 3'UTR with low mutation frequency, thereby exhibiting inhibitory effects. Such results suggest that EV can be used as a general therapeutic agent and/or preventive therapeutic agent with regard to diverse viral infections.

### Example 5. Confirmation of indirect anti-viral effects of EV and miRNA

Viruses infecting the respiratory system, including corona virus, may penetrate cells expressing ACE2 receptor and cause cell damage.

ACE2 receptor is mainly present in the liver, kidneys, male genital tissues, muscle tissues and gastrointestinal, therefore, these organs may be damaged by viruses. In recent clinical studies, a number of patients showed liver damage or organ damage, thus supporting the above opinion.

In the children's hospital in Uhan of China, 6 of 8 COVID-19 patients under treatment in the intensive care unit showed increase in C reactive protein, procalcitonin and dehydrogenated enzymes. Further, 4 of 8 patients had abnormal liver function. Further, cytokine storm was found from 3 of these patients and this symptom was more serious in patients of severe cases.

In another study, 74 of 651 patients (11.4%, average age: 46.14) showed at least one gastrointestinal (GI) symptom such as nausea, vomit or diarrhea, while 10.8% of the patients was confirmed to suffer from liver diseases.

Further, 29 (39.19%) of 74 COVID-19 patients had a high fever. Likewise, 23 patients (31.08%), 8 patients (10.81%) and 16 patients (21.62%) appealed fatigue, dyspnea and headache, respectively.

As expected from the above results, ACE2 receptor was expressed in LL24 (lung fibroblast), BEAS-2B cell (GI epithelia) and liver cell. Further, as demonstrated by Brain Atlas database, ACE2 expression level in the brain was not sufficiently studied. Although the expression level is low, when considering on the basis of the above clinical results, it was predicted that the brain may have a high possibility of becoming a target organ of SARS-CoV-2. Similarly, ACE2 is highly expressed in brain tumor cells and this may suggest that brain cells can become a target of viruses.

Accordingly, in order to confirm indirect anti-viral effects of miRNA derived from different EVs, cells expected to undergo over-immune response by SARS-COV2 such as liver cells, GI cells and brain cells were subjected to experiments.

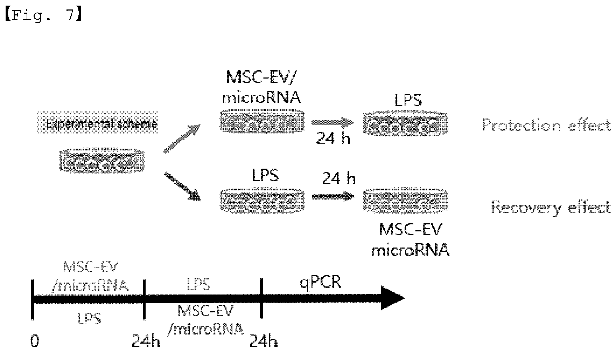

Specific experimental procedures and cell culture methods are as described above. Briefly, the cell line was treated with EV and miRNA, after 24 hours, followed by LPS treatment so as to determine protection effects of EV and miRNA. On the contrary, the cell-line was firstly treated with LPS and, after 24 hours, followed by EV and miRNA treatment so as to determine recovery effects (FIG. 7).

### Example 6. Anti-inflammatory and cytokine storm inhibitory effects of EV and miRNA

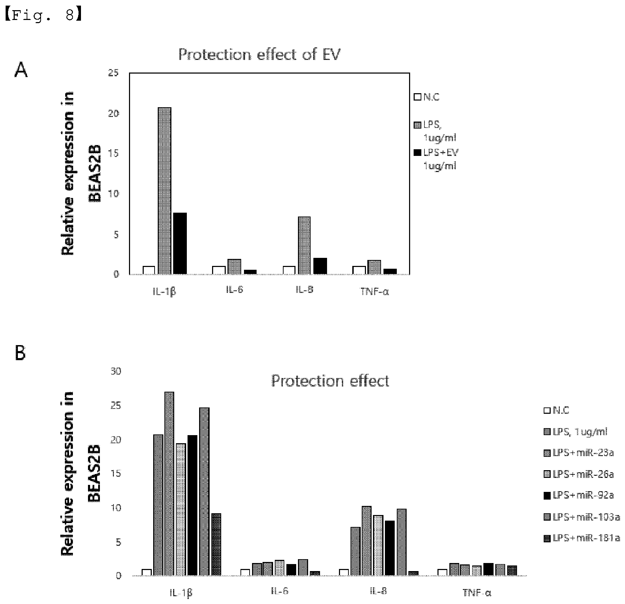

With regard to BEAS-2B (gastrointestinal epithelia), LL24 (lung cell-line), HeoG2 (liver cancer cell-line), MEF (fibroblast), BV2 (microglial cell-line) and M2 (neuron cells), experiments for anti-inflammatory and cytokine storm inhibitory effects of EV and miRNA were implemented with and without LPS and EV treatment. Results of the experiments are shown in FIGS. 8 to 12.

BEAS-2B (gastrointestinal epithelia) was treated with EV, followed by LPS treatment to determine protection effects, and results thereof are shown in FIG. 8a. As a result, it was confirmed that some inflammatory factors, that is, all of IL-1, IL-6, IL-9 and TNF-a showed considerably decreased relative expression, as compared to a case where EV treatment is not involved.

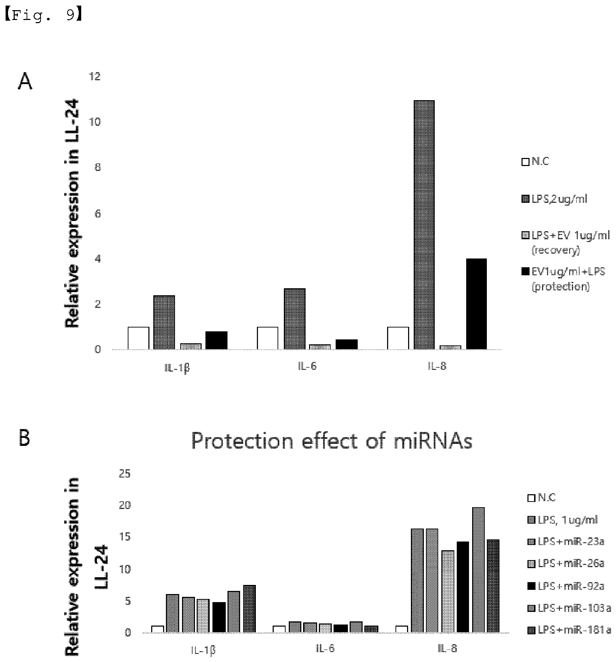

FIG. 9a illustrates results of confirming protection and recovery effects when LL-24 was treated with EV. As a result, it was confirmed that, if treating with EV, all of the inflammatory factors such as IL-1, IL-6 and IL-8 have considerably reduced expression so as to exhibit excellent protection and recovery effects, in particular, very excellent recovery effects.

Further, as shown in FIG. 9b, after treating LL-24 with 5 miRNAs, respectively, protection effects were determined by LPS treatment. As a result, it was confirmed that treatment using miRNA-181a considerably reduced IL-6 expression to the same level as the control.

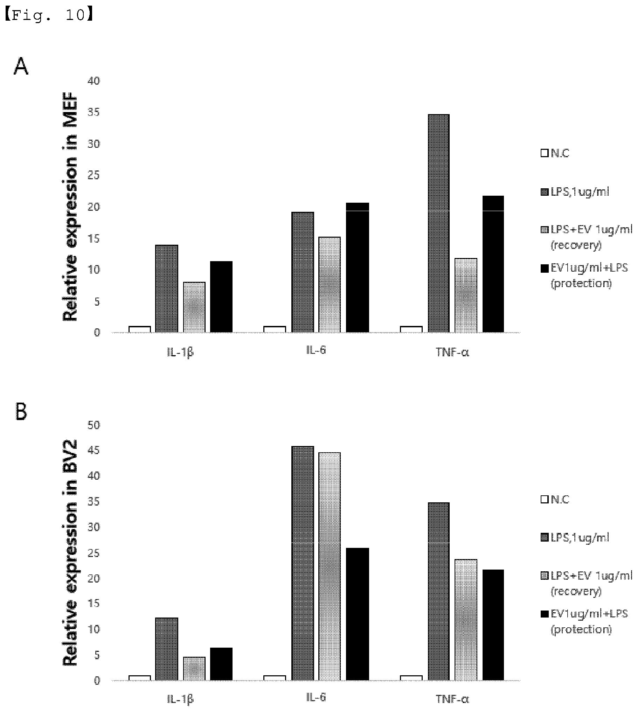

FIG. 10a and 10b illustrate results of MEF (fibroblast) and BV2 (microglial cell-line) protection and recovery effects, respectively. It was confirmed that both of MEF and BV2 showed significantly reduced IL-1, IL-6 and TNF-a as inflammatory factors by EV treatment.

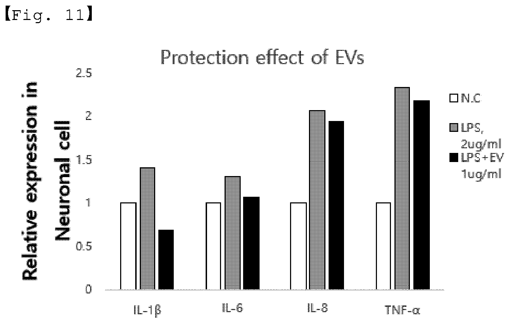

FIG. 11 illustrates results of confirming expression levels of inflammation related factors after EV treatment of M2 as a neuron cell. As a result, it was confirmed that remarkable effects were obtained for only IL-1.

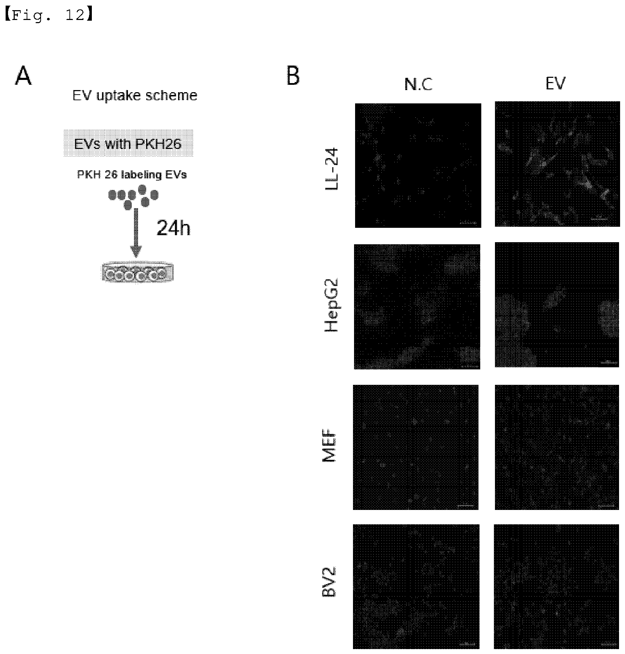

FIG. 12 shows photographed images before and after treatment of each of cell-lines with PKH26-labeled EV. From the images, it could be seen that EV was successfully fused with each cell.

In other words, considering the above results together, it could be seen that EV and miRNA contained EV of the present invention exhibit effects of inhibiting inflammatory factors separately or in combination thereof so as to achieve anti-inflammatory effects, in addition, effects of inhibiting cytokine storm as an over-immune response.

### Example 7. Viral infection treatment effects of EV and miRNA

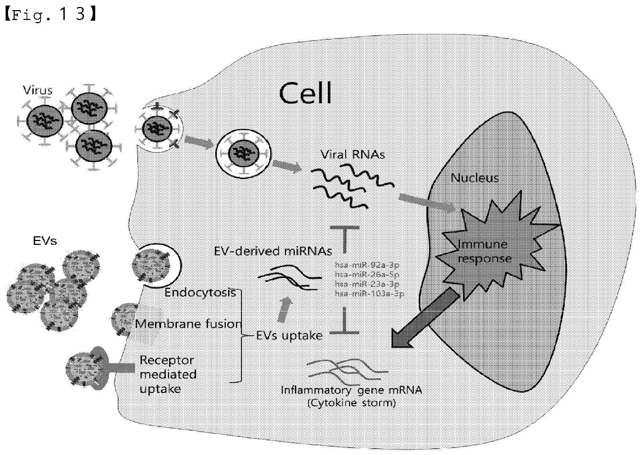

EV and miRNA of the present invention may treat or improve virus and viral infection symptoms through both of direct and indirect routes (FIG. 13).

From the above diverse experiments, the present inventors have found that the placenta, umbilical cord and/or cord blood-derived EVs, placental mesenchymal stem cells (pMSC) obtained from these tissues, and EVs derived therefrom may greatly improve pathological damage of the lungs and, in addition, other type inflammation of the lungs (pneumonia) and viral infection.

In particular, since EV contains regeneration effective factors of the stem cell, this may have a treatment mechanism similar to that of a mesenchymal stem cell (MSC) in treatment of pneumonia, while being considered to be much safer.

Specifically, intravenous administration of MSC may cause embolism and coagulation of blood, whereas EV having a size of 200 to 10⁸ nm is safe much more than MSC, and EV itself is also safer than MSC. Further, EV has advantages such as easy storage, no transformation into malignant or harmful cells, and less possibility of immune response.

Further, results of experiments of the present invention demonstrated that miRNAs contained in tissue-EV, tissue extract EV and pMSC-EV are directly bound to virus genome to inhibit transcription of RNA virus, thereby inhibiting proliferation of the virus genome.

As another important function of EV miRNA, 77 miRNAs existing in diverse EVs target mRNA stimulating immune response so as to exhibit effects of removing RNA transcribed by viruses (*"*viral RNAs*"*), thereby achieving strong indirect effects as well as direct anti-viral effects.

Further, the miRNA of the present invention may be combined with the viral RNA and may also mutually react with pre-inflammatory gene so as to greatly inhibit expression of an inflammatory factor, thereby solving a cytokine storm problem.

Further, miRNA of the present invention may exhibit effects of up controlling IFN-α/β level so as to prevent an IFN-α/β route, thereby inhibiting viral infection avoiding host immune response.

Further, it could be proved that 18 miRNAs of different EVs may directly and mutually react with 3'UTR of SARS-CoV-2, while 5 major miRNAs derived from EV may successfully inhibit SARS-CoV-2.

Some patients infected with corona virus showed liver damage and stomach damage other than main conditions related to the respiratory organ. Among 99 patients in Jinyintan hospital in Uhan, 43 persons had damaged liver function and, in particular, the liver of one patient was seriously damaged. Patients in mild cases are unclear whether liver function was damaged or not, however, patients in serious cases showed a decrease in main liver values, that is, alanine aminotransferase (ALT), aspartate aminotrasferase (AST) and lactic acid dehydrogenase (LDH).

Accordingly, in addition to direct virus inhibitory ability, anti-inflammatory effects of EV derived from the placenta, umbilical cord and/or cord blood may induce synergistic effects. Therefore, when using placenta-derived EV as a therapeutic agent of corona virus, remarkably excellent treatment effects may be achieved.

The above description of the present invention is provided for illustrative purpose, and those skilled in the art, to which the present invention pertains, will understand that the present invention is easily modified into other concrete forms without changing technical spirit or essential features of the present invention. Therefore, the examples described above should be construed as illustrative embodiments without limitation thereof. For example, individual components described in singular form may be separately embodied and some separate components described herein may also be embodied in a combination form.

The scope of the present invention may be defined by the following appended claims, and all alterations or modifications deduced from the meanings and ranges of the claims and equivalent concepts should be construed as being included in the scope of the present invention.

## Claims

1. A cell-free composition, comprising biomolecules extracted from placenta, umbilical cord and/or cord blood along with at least one carrier, excipient or diluents.

2. The cell-free composition according to claim 1, wherein the biomolecules are extracellular vesicles (EV) derived from the placenta and placental mesenchymal stem cell (pMSC).

3. The cell-free composition according to claim 1, wherein the biomolecules are miRNA contained in the cellular vesicles (EV) of the placental mesenchymal stem cell (pMSC).

4. The cell-free composition according to claim 3, wherein the miRNA is one or more selected from the group consisting of hsa-miR-92a-3p, hsa-miR-92b-3p, hsa-miR-181a-5p, hsa-miR-26a-5p, hsa-miR-34a-5p, hsa-miR-23a-3p, hsa-miR-125b-5p, hsa-miR-125a-5p, hsa-miR-103a-3p, hsa-miR-223-3p, hsa-miR-25-3p, hsa-miR-26b-5p, hsa-miR-193a-5p, hsa-miR-1307-3p, hsa-miR-155-5p, hsa-miR-185-5p, hsa-miR-424-3p and hsa-miR-23b-3p.

5. The cell-free composition according to claim 3, wherein the biomolecules included in the cell-free composition are one or more selected from the EV and the miRNA.

6. An anti-viral composition comprising the cell-free composition according to any one of claims 1 to 5.

7. The anti-viral composition according to claim 6, wherein the virus is one or more selected from the group consisting of corona virus, HIV, influenza, entero-virus, porcine reproductive and respiratory syndrome virus, C type hepatitis virus and bovine viral diarrhea virus.

8. The anti-viral virus according to claim 7, wherein the corona virus is corona-19 virus (NMC-nCoVO2) or SARS-CoV-2 virus.

9. A pharmaceutical composition for prevention or treatment of viral infection, comprising the anti-viral composition according to claim 6.

10. The pharmaceutical composition according to claim 9, wherein the viral infection is caused by corona viral infection.

11. A food composition for prevention or improvement of viral infection, comprising the anti-viral composition according to claim 6.

12. An anti-inflammatory composition, comprising the cell-free composition according to any one of claims 1 to 3.
